# EUROPEAN PATENT SPECIFICATION

(11) **EP 2 848 206 B1**
(45) Date of publication and mention of the grant of the patent: **28.11.2018**
(21) Application number: 14184806.9
(22) Date of filing: 15.09.2014
(51) Int. Cl.: A61B 17/122, A61B 17/128

(54) **Surgical clip having compliant portion**
Chirurgische Klammer mit nachgiebigem Abschnitt
Pince chirurgicale ayant une partie souple

(30) Priority: 13.09.2013 US 201314025915
(43) Date of publication of application: 18.03.2015
(73) Proprietor: Ethicon LLC, 00969 Guaynabo (PR)
(72) Inventor: Shelton, IV Frederick, Hillsboro, OH 45133 (US)
(74) Representative: Carpmaels & Ransford LLP

(56) References cited:
- EP-A1- 1 547 528
- US-A- 5 843 101
- US-A1- 2006 235 468
- US-A1- 2008 132 915
- US-A1- 2009 187 198

## Description

### FIELD OF THE INVENTION

The present invention relates to surgical instruments and in particular to surgical clips.

### BACKGROUND OF THE INVENTION

During many surgical procedures, the surgeon will have to close or ligate various blood vessels and other ducts before severing them in order to prevent excessive bleeding, and reduce the risk of other complications to the patient. One ligation technique is to tie a suture about the vessel to close the vessel. Alternatively, a surgeon can place a clip having a pair of legs connected at their proximal ends about the vessel, and urge or squeeze the legs together to close the vessel.

One drawback associated with some current clips used for ligating vessels is that the legs of the clip may tend to separate to some extent following release from a clip applier. This phenomenon is called duck-billing. Duck-billing can result in insufficient ligation of a vessel, thus leading to excessive blood loss and/or unnecessary damage to the vessel. Further, some known ligation clips are often difficult to preload into a clip applier because of resistance between the tissue disposed between the jaws and the gripping features on the clip legs.

Accordingly, there remains a need for an improved surgical instrument and method, and in particular for surgical clips used for ligating blood vessels, other ducts, and the like.

US2006235468 describes a clip which can be used for ligating tissue, such as vessels, other tubular ducts, and the like. The clip has opposed first and second leg members having proximal and distal ends. The proximal end of each leg member is connected by an apex having a notch formed therein. Moreover, each leg member has an inner tissue-contacting surface and an outer compression-receiving surface, both of which include features to provide a more secure ligation of the vessel or duct.

US2008132915 describes devices for achieving hemostasis and leakage control in hollow body vessels such as the small and large intestines, arteries, and veins as well as ducts leading to the gall bladder and other organs. The devices utilize removable or permanently implanted, broad, soft, parallel jaw clips with minimal projections to maintain vessel contents without damage to the tissue comprising the vessel.

US5843101 describes a disposable clip for the temporary occlusion of blood vessels, bowels, and ducts. A pair of substantially rigid, spaced-apart members are joined by way of a malleable hinge. Each member includes an inwardly oriented surface which oppose and face one another and outwardly oriented surfaces which face away from one another. A layer of compressible material is supported against the inwardly oriented surface of each member and, with an externally applied pressure exerted upon the outwardly oriented surfaces, the pair of rigid members are urged toward one another, thereby compressing and at least temporarily occluding the vessel between the layers of compressible material, with the malleable hinge maintaining the clip in this configuration until removal.

### SUMMARY OF THE INVENTION

Aspects of the present disclosure may be described by the term "embodiment" for brevity, however, the present invention is defined by the independent claim with further details of specific embodiments being provided by the dependent claims.

### BRIEF DESCRIPTION OF THE DRAWINGS

The invention will be more fully understood from the following detailed description taken in conjunction with the accompanying drawings, in which:
FIG. 1 is a perspective view of a surgical clip disclosed herein;
FIG. 2A is a side perspective view of a clip;
FIG. 2B is a side perspective view of a portion of the distal end of a leg member of the clip of FIG. 2A;FIG. 2C is a plan view of the clip of FIG. 2A;FIG. 2D is a sectional view of the clip of FIG. 2C along the lines 2D-2D;FIG. 2E is a sectional view of the clip of FIG. 2C along lines 2E-2E;FIG. 3 is another perspective view of a clip.FIG. 4A is a perspective view of a clip.
FIG. 4B is a top plan view of an inner portion of the apex of the clip of FIG. 4A;FIG. 4C is a side perspective view of an inner portion of the apex of the clip of FIG. 4A;FIG. 5A is another side perspective view of a clip in an open position;FIG. 5B is a side perspective view of the clip of FIG. 5A in a first state of partial closure;FIG. 5C is a side perspective view of the clip of FIG. 5A in a state of almost full closure;FIG. 5D is a side perspective view of the clip of FIG. 5A fully closed;FIG. 5E is a side perspective view of the clip of FIG. 5A following release by a clip applier;
FIG. 6 is a side view of a clip having a compliant element in accordance with the invention;
FIG. 7 is a side view of a clip without a compliant element in a state of full closure;FIG. 8 is a side view of the clip of FIG. 6 in a state of full closure;FIG. 9 is a side view of a clip having a compliant element comprising a plurality of ribs in accordance with the invention;
FIG. 10 is a perspective view of a distal end of one leg of a clip having a ribbed compliant element in accordance with the invention; and
FIG. 11 is a side view of a clip having a ribbed compliant element in accordance with the invention in a state of full closure.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments.

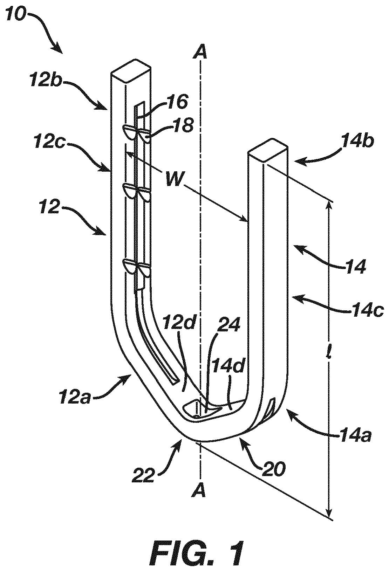

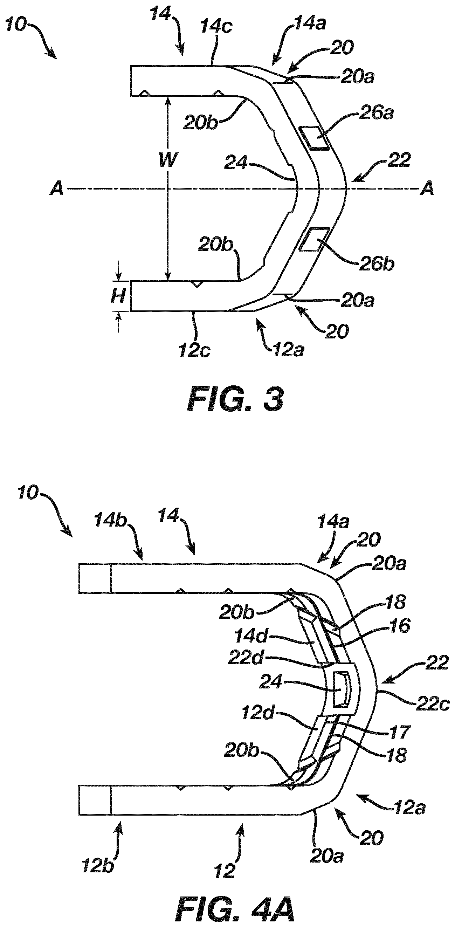

The present invention provides various devices for ligating tissue, such as vessels, other tubular ducts, and the like. FIGS. 1-4C illustrate exemplary embodiments not forming part of the invention of a clip disclosed herein in an open position. Referring generally to FIG. 1, the clip 10 in its open position is generally U-shaped having opposed leg members 12, 14 joined at an apex 22. Each leg member 12, 14 has a knee portion 20 disposed distally of the apex 22. Moreover, each leg member 12, 14 has an inner tissue-contacting surface 12d, 14d and an opposed outer surface 12c, 14c, both of which may have features to provide a more secure ligation of the vessel or duct. For example, the inner surface(s) 12d, 14d can include various tissue-grasping elements formed therein (discussed in more detail below). The outer surface(s) 12c, 14c can have at least one raised area 26 (shown in FIG. 3) formed thereon between the knee portion 20 and the apex 22. While clip 10 is described herein in the context of a device to ligate vessels, one skilled in the art will appreciate that the surgical clip 10 can be used to ligate a variety of other body tissues, including but not limited to, veins, arteries, ducts, or any other tubular member within a patient for which ligation is desired. Moreover, the clip 10 can be used in a variety of clip appliers, thereby effecting a wide range of surgical procedures. Although the clip 10 is described herein with respect to ligation, it is understood that a variety of other applications are possible as well.

The clip 10 can have any shape in its open configuration that allows it to effectively ligate a vessel, such as a substantially U-shaped or a substantially V-shaped design. As noted above, in an exemplary embodiment, the clip 10 is substantially U-shaped. That is, proximal portions 12a, 14a of the leg members 12, 14 of the clip 10 are oriented at an acute angle with respect to the central axis A of the clip 10, and transition at a knee portion 20, to an orientation where distal portions 12b, 14b of the leg members 12, 14 are parallel with respect to one another and to central axis A.

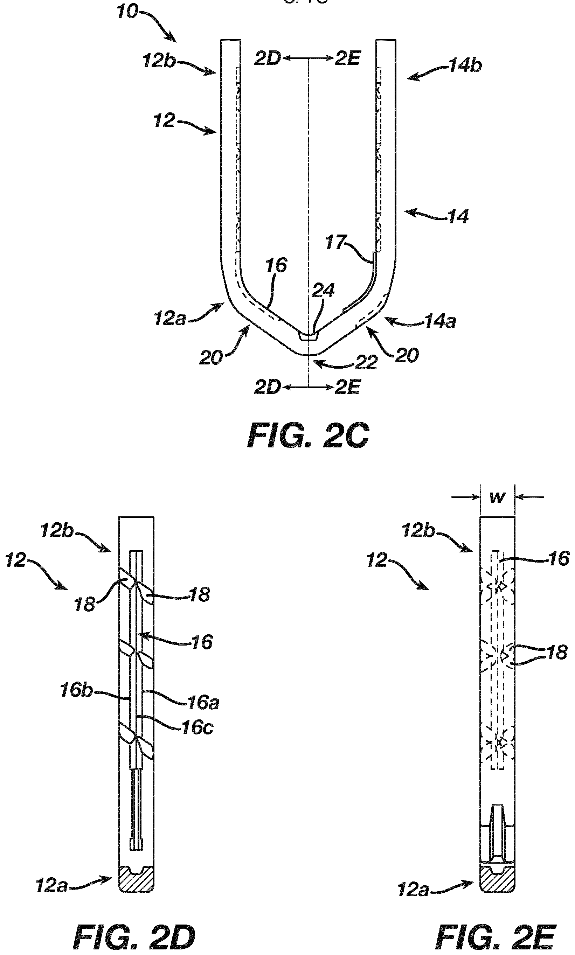

One skilled in the art will appreciate that the size of the clip 10 can vary depending upon its particular application. In an exemplary embodiment, the clip 10 can have a length / in the range of about 5 mm to 15 mm, and more preferably in the range of about 7.5 mm to 8.5 mm. In its open configuration, the clip 10 can have a width W as shown in FIG. 3 measured between opposed inner surfaces 12d, 14d of the leg members 12, 14 in the range of about 2 mm to 8 mm, and more preferably in the range of about 3 mm to 4 mm. The size of the leg members 12, 14 can also vary depending upon the particular application, however in one embodiment, each leg member 12, 14 can have a width w, shown in FIGS. 2D and 2E, less than 1.27 mm (0.050 inch), more preferably in the range of about 0.635 mm (0.025 inch) to about 1.016 mm (0.040 inch), most preferably less than about 0.889 mm (0.035 inch). Moreover, each leg member 12, 14 can have a height H (shown in FIG. 3) in the range of about 0.381 mm (0.015 inch) to 0.762 mm (0.030 inch), and more preferably in the range of about 0.4572 mm (0.018 inch) to 0.635 mm (0.025 inch), and most preferably in the range of about 0.4826 mm (0.019 inch) to 0.508 mm (0.020 inch).

The clip can also have physical properties, such as yield strength, that are appropriate for a desired application. In an exemplary embodiment, the yield strength is greater than about 193 MPa (28 ksi) and less than about 413.7 MPa (60 ksi), and more preferably in the range of about 206.8 MPa (30 ksi) to 344.7 Mpa (50 ksi). In general, clip 10 can have a yield strength that is equivalent to or greater than clips having larger dimensions.

Clip 10 is further designed so that, upon closure, a vessel, for example, is completely encased between the leg members 12, 14 of the clip 10. This is done by urging the leg members 12, 14 of the clip 10 together, typically with the assistance of an applier, to surround the vessel.

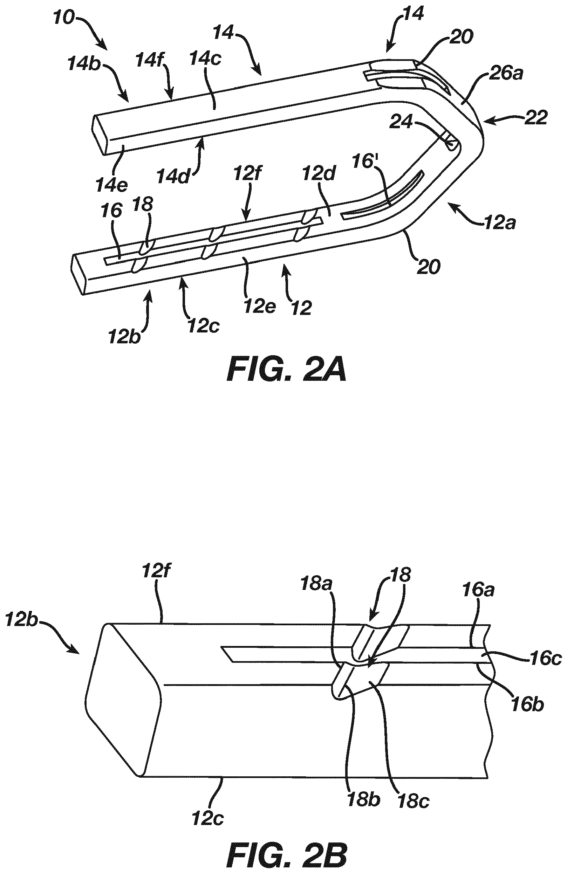

Referring now to FIGS. 2A-2E, the clip 10 has opposed first and second leg members 12, 14 each having proximal and distal ends 12a, 14a, 12b, 14b. The proximal and distal ends 12a, 14a, 12b, 14b have opposed inner tissue-contacting surfaces 12d, 14d and outer compression-receiving surfaces 12c, 14c that are connected by superior and inferior sides 12e, 14e, 12f, 14f. One skilled in the art will appreciate that the leg members 12, 14 can have any cross-sectional shape that allows them to effectively close and engage tissue, such as a vessel. Exemplary cross-sectional shapes include, but are not limited to, triangular, rectangular, trapezoidal, and pentagonal. As shown, however, the leg members 12, 14 are substantially rectangular. The substantially rectangular leg shape is believed to provide an optimized design that includes a greater bending resistance for a given clip leg space envelope.

The leg members 12, 14 can also have a variety of features formed therein or thereon to assist with the ligation of a vessel or duct. For example, the inner surface 12d, 14d of each leg member 12, 14 can include tissue-grasping elements, and the outer surface 12c, 14c of each leg member 12, 14 can include a knee portion 20 as well as at least one raised area 26. Optionally, one or more grooves may be formed on the outer surface 12c, 14c as well.

As shown in FIGS. 2A-2E, the tissue-grasping elements formed on an inner surface 12d, 14d of each leg member 12, 14 can include both primary 16, 17 and secondary 18 tissue-grasping elements. The primary tissue-grasping elements 16, 17 can have any configuration that allows them to effectively hold a vessel or duct. In one embodiment, the primary tissue-grasping elements can include at least one tongue 17 formed on the inner surface 14d of the second leg member 14 and at least one groove 16 formed on the inner surface 12d the first leg member 12. The groove 16 and tongue 17 can extend continuously along the inner surface 12d, 14d of each leg member 12, 14. Alternatively, the inner surface 12d, 14d can include multiple groove 16 and tongue 17 segments formed therein.

The groove 16 and tongue 17 can be formed in a variety of locations on each of the first and second leg members 12, 14. In one embodiment, the groove 16 and tongue 17 can extend longitudinally along the entire length or along at least a portion of the length of the inner surface 12d, 14d of each respective leg member 12, 14. Alternatively, the groove 16 and tongue 17 can extend from the distal end 12b, 14b of each leg member 12, 14 to just distal from the apex 22, or from the distal end 12b, 14b of each leg member 12, 14 to just distal to the knee portion 20. Moreover, the groove 16 and tongue 17 can extend distally from the apex 22 to a position just distal to the knee portion 20.

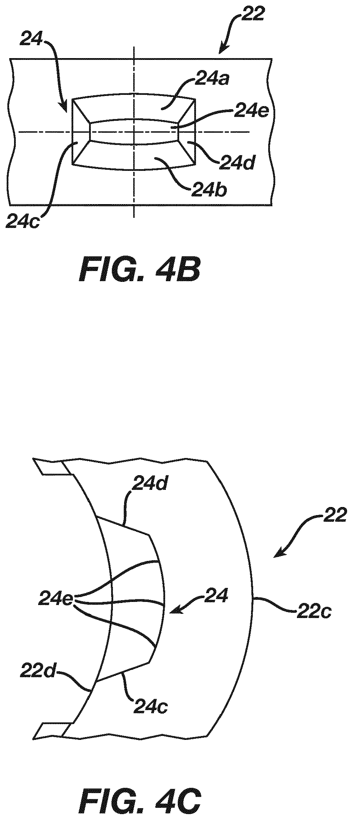

By way of non-limiting example, FIG. 1 illustrates a longitudinal groove 16 and a longitudinal tongue 17 that extend through the knee portion 20 and terminate just distal to the notch 24 in the apex 22. Alternatively, FIG. 2A illustrates a longitudinal groove 16 and a longitudinal tongue 17 that extend from the distal end 12b, 14b of each leg member 12, 14 to a position just distal to the knee portion 20. A second longitudinal groove 16' and longitudinal tongue 17' combination is then formed just distal to the knee portion 20, extending just distal to the apex 22. Moreover, FIG. 4A illustrates a longitudinal groove 16 and a longitudinal tongue 17 that are formed along the entire inner surface 12d, 14d of each of the first and second leg members 12, 14. The groove 16 and tongue 17 combination shown in FIG. 4A terminates in the notch 24 of the apex 22, as will be discussed in more detail below.

The tongue 17 and groove 16 can be disposed so as to be complementary to one another. Alternatively, the tongue 17 and groove 16 can be located at different locations along each respective leg member 12, 14. In an exemplary embodiment, the tongue 17 are groove 16 are complementary and disposed opposite one another, such that once the clip 10 is applied to a vessel the tongue 17 will urge the tissue of the walls of blood vessel into the corresponding juxtaposed groove 16. This cooperation between the tongue 17 and the groove 16 inhibits longitudinal and angled dislocation of the clip 10 relative to the vessel, and it also effectively reduces the gap between the inner (tissue contacting) surfaces of each respective leg member 12, 14.

One skilled in the art will appreciate that the groove 16 can have a variety of shapes. In an exemplary embodiment, the groove 16 is complementary in shape to the tongue 17 and can be hemispherical, rectangular, triangular, trapezoidal, or oblong. As shown in FIG. 2B, an exemplary embodiment uses a groove 16 that is somewhat triangular, having opposed sidewalls 16a, 16b connected by a base portion 16c. The sidewalls 16a, 16b can be oriented at various angles with respect to the inner surface 12d, 14d of the leg members 12, 14. In one embodiment, the sidewalls 16a, 16b are oriented at an angle less than 120 degrees relative to the inner surface 12d, 14d of the leg members 12, 14, and more preferably at an angle less than 110 degrees relative to the inner surface 12d, 14d of the leg members 12, 14.

One skilled in the art will appreciate that the base portion 16c can have a variety of configurations. For example, the base portion 16c can be planar or slightly rounded. In an exemplary embodiment, however, the base portion 16c is slightly rounded.

One skilled in the art will appreciate that the groove 16 should be of dimensions that are effective to ligate tissue. For example, the groove 16 can have depths in the range of about 0.0381 mm (0.0015 inch) to 0.1778 mm (0.007 inch), more preferably, in the range of about 0.0635 mm (0.0025 inch) to 0.1016mm (0.004 inch). In one exemplary embodiment, the groove 16 can have a depth of about 0.0635 mm (0.0025 inch). Further, groove 16 can have a width in the range of about 0.1016 mm (0.004 inch) to 0.508 mm (0.020 inch), more preferably in the range of about 0.1524 mm (0.006 inch) to 0.3302 mm (0.013 inch). Moreover, the width of the groove 16 can be uniform throughout the length of the groove 16, or it can decrease in the proximal or distal direction. In an exemplary embodiment, the groove 16 has a uniform width.

One skilled in the art will also appreciate that the tongue 17 can also have a variety of configurations. However, in an exemplary embodiment, the tongue 17 is complementary in shape and size to the groove 16. Thus, the tongue 17 can be hemispherical, rectangular, triangular, trapezoidal, or oblong. In an exemplary embodiment, the tongue 17 is substantially rectangular or trapezoidal.

The tongue 17 can also vary in size, however in an exemplary embodiment, the tongue 17 has a size that is complementary to the size of the groove 16, with a height and a width no greater than, and preferably slightly less than, the dimensions of the groove 16. This provides room for the vessel tissue and minimizes shearing action and locally excessive pressures on the vessel tissue during clip forming. That is, the tongue 17 can have a height in the range of about 0.0381 mm (0.0015 inch) to 0.1778 mm (0.007 inch), more preferably in the range of about 0.0635 mm (0.0025 inch) to 0.1016 mm (0.004 inch). In one exemplary embodiment, the tongue 17 can have a height of about 0.0635 mm (0.0025 inch). The tongue 17 can also have a width in the range of about 0.1016 mm (0.004 inch) to 0.508 mm (0.020 inch), more preferably in the range from about 0.1524 mm (0.006 inch) to 0.3302 mm (0.013 inch). Moreover, and also similar to the groove 16 above, the tongue 17 can have a uniform width or a width that decreases in the proximal or distal direction. In an exemplary embodiment, the tongue 17 has a uniform width.

In addition to primary tissue-grasping elements 16, 17, the inner surfaces 12d, 14d of each of the first and second leg members 12, 14 can have at least one secondary tissue-grasping element 18, as shown in FIG. 2B. While in one embodiment the secondary tissue-grasping elements 18 are formed on the inner surfaces 12d, 14d of both the first and second leg members 12, 14, the secondary tissue-grasping element 18 can optionally be formed on the inner surface 12d, 14d of only one of the first and second leg members 12, 14. One skilled in the art will appreciate that the inner surfaces 12d, 14d of the first and second leg members 12, 14 can have any number of secondary tissue-grasping elements 18. In the exemplary embodiment, the inner surface 12d, 14d has at least four secondary tissue-grasping elements 18.

The secondary tissue-grasping elements 18 can have any configuration that allows them to grasp tissue following application of the clip 10 to the vessel or duct. As shown in FIG. 2B, exemplary secondary tissue-grasping elements 18 are in the form of channels having opposed first and second walls 18a, 18b connected by base wall 18c. The channels are generally saw-toothed in shape, however can also be undercut. In an exemplary embodiment, the first wall 18a is formed at an acute angle relative to the inner surface 12d, 14d of each leg member. In an exemplary embodiment the angle is in the range of about 40 degrees to 90 degrees, and more preferably the angle is about 75 degrees. The second wall 18b is likewise oriented at an acute angle relative to the inner surface 12d, 14d of each leg member. The acute angle of the second wall 18b, which is generally shallower than the angle of the first wall 18a, can be in the range of about 15 degrees to about 75 degrees, and more preferably it is about 45 degrees. One skilled in the art will appreciate that the walls 18a, 18b, 18c can be straight or arcuate, but in the exemplary embodiment the walls 18a, 18b, 18c are slightly arcuate to facilitate grasping.

As shown in FIGS. 2D-2E, the secondary tissue-grasping elements 18 extend across the width w of the first and second leg members 12, 14 at an angle (e.g., about 45 degrees) relative to a longitudinal axis of the leg members 12, 14. In an exemplary embodiment, one segment of the secondary tissue-grasping element 18 is located on one side of the tongue 16 or groove 17 on the first leg member 12, and a second segment 18 continues at the same angle on the other side of the tongue 16 or groove 17. The secondary tissue-grasping elements 18 are similarly constructed on the second leg member 14, however they are angled at an orientation opposite that of the first leg member 12. Thus, when the leg members 12, 14 close around a vessel or duct, they form a superimposed "x," as shown in FIG. 2E. This configuration allows for a greater percentage of the tissue to be grasped by the secondary tissue-grasping elements 18, thereby resulting in more effective ligation.

The leg members 12, 14 can have any number of secondary tissue-grasping elements 18 formed thereon. In the exemplary embodiment, however each leg member 12, 14 has three secondary tissue-grasping elements 18 formed thereon. One skilled in the art will appreciate that the secondary tissue-grasping elements 18 can be uniformly or non-uniformly spaced apart from one another. In an exemplary embodiment, the secondary tissue-grasping elements 18 are uniformly spaced apart from one another at a distance in the range of about 1.27 mm (0.050 inch) to 2.032 mm (0.080 inch).
Moreover, the secondary tissue-grasping elements 18 can have any size and depth that is effective to engage and maintain contact with tissue. However, in an exemplary embodiment, the secondary tissue-grasping elements 18 are sized in the range of about 0.2032 mm (0.008 inch) to 0.3048 mm (0.012 inch) wide by about 0.0381 mm (0.0015 inch) to 0.0889 mm (0.0035 inchs) deep.

One skilled in the art will appreciate that the leg members 12, 14 of the exemplary clip 10, as shown in FIGS. 1-4C, can include any combination of primary tissue-grasping elements 16, 17 and secondary tissue-grasping elements 18. An exemplary clip 10, however, includes both primary and secondary tissue-grasping elements 16, 17, 18. In another exemplary embodiment (not shown), the inner surface 12d, 14d of the leg members 12, 14 can be smooth and free of primary and secondary tissue-grasping elements. The structure and closing properties of the clip 10, as discussed herein, allow adequate tissue ligation without the need for any type of tissue-grasping elements formed on the inner surface 12d, 14d of the leg members 12, 14.

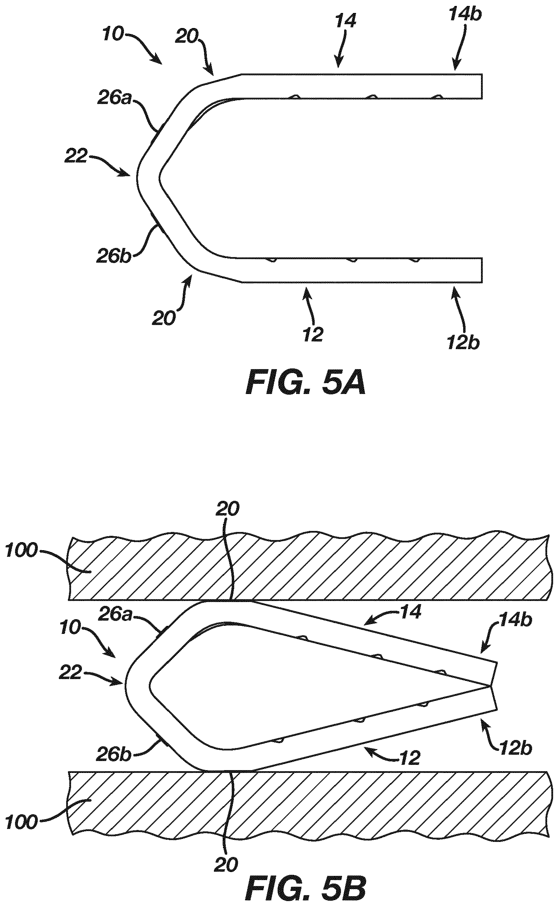

As shown, for example, in FIG. 3, the outer surface 12c, 14c of each leg member 12, 14 can include a bend or knee portion 20. The knee portion 20 allows the leg members 12, 14 to transition from being acutely angled relative to the central axis A of the clip 10 to being substantially parallel relative to one another and to the central axis A of the clip 10. The angled knee portions 20 of the leg members 12, 14 can be formed at a variety of angles relative to the central axis A of the clip 10, however in an exemplary embodiment the angle can be in the range of about 45 degrees to about 65 degrees. In one embodiment, the knee portion 20 is designed so as to be parallel to the force applying jaws of a clip applier during a part of the clip closing process as shown in FIG. 5B. This construction is believed to enhance clip retention by the clip applier during deployment.

The knee portion 20 can have a variety of configurations to effect the transition of the leg members 12, 14, however an exemplary knee portion 20 has a beveled or flattened outer surface 20a and an arcuate inner surface 20b. The bevel on the outer surface 20a can extend over any length sufficient to effect the transition, however in an exemplary embodiment the bevel is in the range of about 0.762 mm (0.030 inch) to 1.27 mm (0.050 inch). The outer surface 20a of the knee portion 20 can optionally include a groove (not shown) formed therein to facilitate formation of a raised tongue 17 on the inner surface 12d, 14d of the leg members 12, 14. The groove can be similar in shape
and size to the longitudinal groove 16, discussed herein with respect to FIGS. 2A-2E. The inner surface 20b of the knee portion 20 can also optionally include features to assist with the ligation of the vessel, duct, or tissue. For example, the inner surface 20b can include primary and/or secondary tissue-grasping elements 16, 17, 18 similar to those discussed above with respect to FIGS. 2B-2D.

As noted above, the outer surface 12c, 14c of each leg member 12, 14 can have features to help provide a more secure occlusion and clip performance. In one embodiment, shown in FIG. 3, a raised area 26 extends over a portion of the width of the leg members 12, 14 that is slightly proximal to the knee portion 20. In an exemplary embodiment, the raised area 26 is located approximately one-third of the way between the apex 22 and the knee portion 20, closer to the apex 22. The raised portion 26 is believed to help to reduce overbending of the knee 20 as well as to help maintain the legs 12, 14 of the clip 10 together after the clip 10 is fully closed. While FIG. 3 shows the raised area 26 formed on both the first and second leg members 12, 14, in alternate embodiments, the raised area 26 can be formed on either the first leg member 12 or the second leg member 14. Moreover, the outer surface 12c, 14c of each leg member 12, 14 can have any number of raised areas 26. In the exemplary embodiment, the outer surface 12c, 14c of each leg member 12, 14 has one raised area 26a, 26b.

The raised area 26a, 26b can have any shape that allows the effective application of compressive force to the apex 22 such that the apex 22 is crimped to a greater degree than the knee portion 20. That is, the raised area 26a, 26b is believed to allow the region of the leg member 12, 14 between the apex 22 and the knee 20 to be more elastic, enabling the knee portion 20 to spring back to a small degree while maintaining adequate contact between the distal ends 12b, 14b of the leg members 12, 14. In an exemplary embodiment, the raised area 26a, 26b is a pad having a shape that is complementary to the shape of the leg member 12, 14. Thus, the raised area 26a, 26b can be triangular, rectangular, trapezoidal, pentagonal, etc., but in an exemplary embodiment, the raised area 26a, 26b is substantially rectangular.

One skilled in the art will appreciate that the raised area 26a, 26b can have a variety of sizes, depending upon whether full closure or partial closure of the clip is desired. By way of non-limiting example, if full closure of the clip is desired, the height of the raised area 26a, 26b should be able to maintain the preload at the distal tips of the leg members 12, 14. In an exemplary embodiment, the raised area 26a, 26b has a height in the range of about 0.0127 mm (0.0005 inch) to 0.0635 mm (0.0025 inch), and more preferably is about 0.0254 mm (0.001 inch). The raised area 26a, 26b can also have a length that is large enough so that it can adequately sustain the applied pressure from a clip applier. In an exemplary embodiment, the raised area 26a, 26b can have a length of about 0.508 mm (0.020 inch), and a width of about 0.254 mm (0.010 inch). If partial closure of the clip is desired, the height of the raised area 26a, 26b can be increased.

As noted above, the proximal ends of each of the leg members 12a, 14a are connected to one another by an apex 22. While the apex 22 can have a variety of shapes, as shown in FIGS. 4A-4C, the apex 22 is substantially U-shaped or substantially V-shaped, and has opposed inner (tissue-contacting) 22d and outer (non-tissue contacting) faces 22c that are connected by superior and inferior surfaces (not shown).

The inner surface 22d of the apex 22 can have a variety of configurations in order to assist with ligation, for example, at least one notch 24 can be formed therein. While the inner surface 22d can have any number of notches formed therein, an exemplary embodiment utilizes one notch 24. One skilled in the art will appreciate that the notch 24 can have any configuration that allows for the ligation of tissue. In an exemplary embodiment, the notch 24 is formed in a U-shaped channel that extends through the inner surface 22d of the apex 22. The U-shaped channel may join the tongue 16 and groove 17 that extend along at least a portion of length of the inner surface 12d, 14d of the leg members 12, 14.

The notch 24 can further have a variety of shapes to optimize its mechanical properties and make it stiff and strong for the amount of material in it, yet leaving open space for the material in compression on the inner side of the clip 10 to flow into during the plastic deformation that occurs during clip formation. In an exemplary embodiment, as shown herein, the notch 24 is substantially trapezoidal. That is, as shown in FIGS. 4B-4C, the notch 24 has opposed first and second walls 24a, 24b connected by opposed third and fourth walls 24c, 24d with a base portion 24e extending therebetween. While the walls 24a, 24b, 24c, 24d can have a variety of configurations, in an exemplary embodiment the walls 24a, 24b, 24c, 24d are formed at an acute angle relative to the inner surface 22d of the apex 22. The angle can be any acute angle, but it is preferably in the range of about 75 degrees. One skilled in the art will appreciate that the walls 24a, 24b, 24c, 24d, 24e can have also have any shape that provides an area into which deformed tissue can flow. As shown, the walls and the base portion 24a, 24b, 24c, 24d, 24e are rounded or slightly contoured.

The notch 24 can have a variety of sizes and depths, perhaps best described in relationship to the thickness and width of the clip leg members 12, 14. The width of notch 24 should be such that the webs of material at apex surface 22d are in the range of about 0.127 mm (0.005 inch) to 0.254 mm (0.010 inch) wide. The depth of notch 24 should be in the range of about 30 percent to 60 percent of the distance between apex surfaces 22c and 22d, with an exemplary range of about 30 percent to 40 percent of the distance between surfaces 22c and 22d. The length of notch 24 should be in the range of about 1 times to 2 times the thickness of the clip leg members 12, 14, with an exemplary length in the range of about 1.1 times to 1.4 times the thickness of the clip leg members 12, 14. In the case of larger, wider clips, optimum results might require the use of two or more notches in order to maintain the webs of material at surface 22d in the range of about 0.127 mm (0.005 inch) to 0.254 mm (0.010 inch). Other aspects of multiple notches would be expected to follow the guidelines listed above.

The outer face 22c of the apex 22 can also have a variety of configurations in order to assist with ligation. In an exemplary embodiment, the outer face of the apex 22c has two opposed beveled surfaces that meet in a rounded tip. The outer face 22c of the apex 22 is not sharply formed, but rather has a fabrication-induced radius, thereby allowing for a more secure ligation.

The clip 10 disclosed herein can be made from a variety of surgically-appropriate materials including metals and polymers. Moreover, the material can be a bioabsorbable material or a non- bioabsorbable material. In one embodiment, the clip 10 can be made of a metal or a metal alloy having a relatively high annealed state yield strength and a relatively high strain hardening rate, in comparison to existing ligation clips. Suitable metals include tantalum, titanium, stainless steel, or alloys thereof. By way of non-limiting example, the clip 10 can be made from commercially pure titanium or ASTM grade CP1 titanium. This material, when compared with conventional materials, is able to be strain hardened to a greater extent without causing excessive gaps in the formed clip 10.

Moreover, a small amount of interstitial elements, such as oxygen or nitrogen, can be added to the clip material to maintain the formability of the clip 10. In an exemplary embodiment, oxygen can be incorporated within the clip material. Other interstitial elements can include nitrogen, carbon, and iron. The clip 10 can also optionally be coated with an antimicrobial or antibiotic material in order to increase the effectiveness of the clip against a broad range of infectious agents or pathogens.

FIGS. 5A-5E sequentially illustrates selected steps of clip closure, for example to ligate a vessel. As shown in FIG. 5A, an open clip 10 is presented, and it can be placed around a desired vessel. A closing force is then applied to the outer surface 12c, 14c of the leg members 12, 14 by, for example, the force-applying jaws 100 of a clip applier. As clip closure begins, as shown in FIG. 5B, the knee portion 20 and the apex 22 are deformed such that the distal ends 12b, 14b of the leg members 12, 14 are moved inward towards one another. In the position shown in FIG. 5B, the clip features at the knees 20 have become predominately parallel to each other and to the clip applying jaws 100, helping to stabilize the clip 10 in the jaws 100 of the applier.

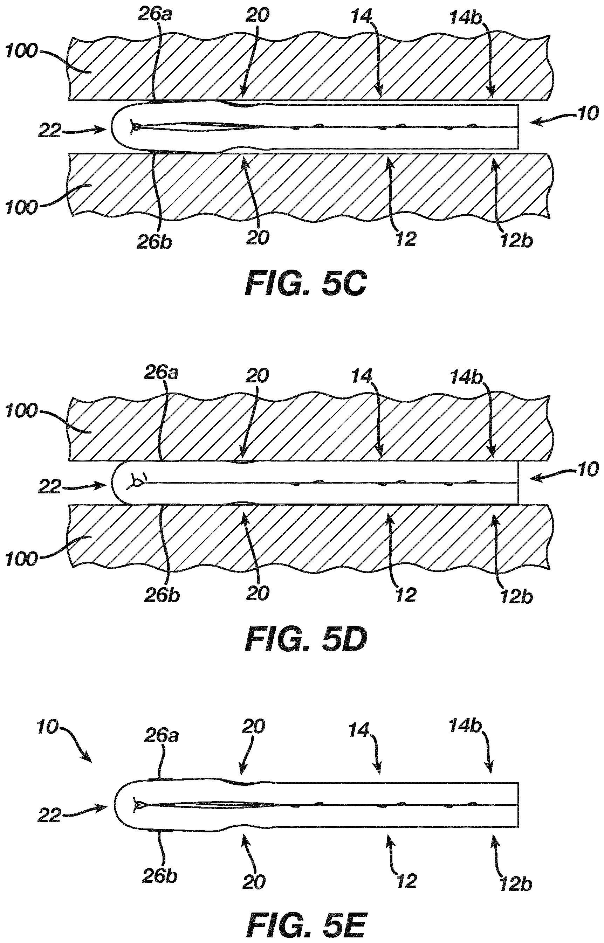

As the application of closing force to the clip 10 continues and the distal ends 12b, 14b of the leg members 12, 14 move closer to one another, the raised area 26 begins to share the clip radial closure forces with the knee portion 20. As a result of this reduction in pressure, the knee 20 is deformed to a lesser extent, as shown in FIG. 5C. FIG. 5D illustrates a condition of full clip closure, with the closing force still applied to the clip 10 by the closing jaws 100. At the final stages of crimping, the raised area 26a, 26b takes some load off of the knee portion 20, thereby reducing the amount of plastic deformation of the knee portion 20. The raised area 26 thus allows the knee portion 20 to have increased elasticity, such that, for example, the knee portion 20 can bend inward slightly when forming loads are released, preloading the tips of the clip 10. This is particularly advantageous in that when the applier is removed from the clip 10 as shown in FIG. 5E, the raised area 26 allows the leg members 12, 14 to remain together from the knee portion 20 to the distal ends 12b, 14b thereof, thereby lessening the duck-billing of the clip 10.

One advantage provided by clip 10 is that it tends to be more resistant to "duck-billing," a condition in which the distal tips of the leg members 12, 14 of the clip 10 tend to separate after the closing force is removed. Some previously known clips tend to duckbill as a result of residual elasticity within the apex. Clip 10 is believed to overcome the tendency to duckbill because the apex 22 is able to crimp to a greater extent and thus minimize the effect of any springback. At the same time, increased elasticity between the apex 22 and the knee portion 20 enables any springback at the knee portion 20 to direct the distal ends 12b, 14b of the leg members 12, 14 toward each other. An additional advantage of the above-mentioned characteristics of the clip 10, is that tissue is able to be captured at any location within the clip 10, including near the apex 22 or near the distal ends 12b, 14b of the leg members 12, 14, and still be effectively ligated. As a result, a surgeon can securely ligate vessels having a variety of sizes.

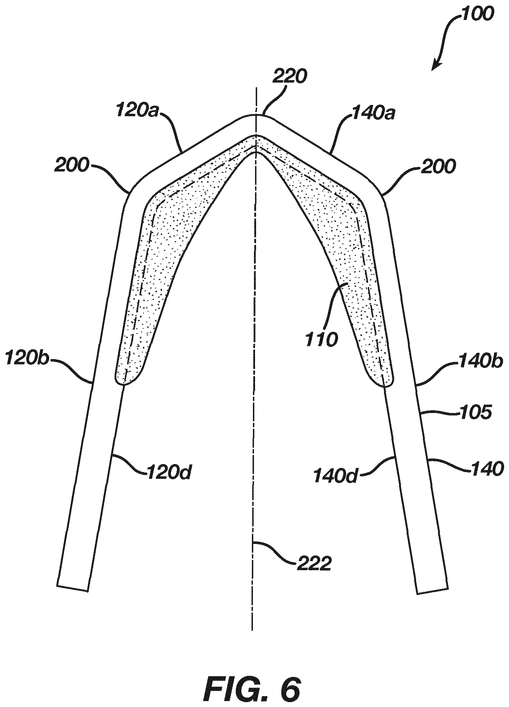

FIG. 6 illustrates an exemplary embodiment according to the invention of a clip 100 having a compliant portion 110 FIG. 6 illustrates clip 100 in the open position. Clip 100 in its open position is generally U-shaped having opposed leg members 120, 140 joined at an apex 220 and arranged about a centerline 222. Each leg member 120, 140 has a knee portion 200 disposed distally of the apex 220. Moreover, each leg member 120, 140 has an inner surface 120d, 140d and an opposed outer surface 120c, 140c. While clip 100 is described herein in the context of a device to ligate vessels, one skilled in the art will appreciate that surgical clip 100 can be used to ligate a variety of other body tissues, including but not limited to, veins, arteries, ducts, or any other tubular member within a patient for which ligation is desired. Moreover, clip 100 can be used in a variety of clip appliers, thereby effecting a wide range of surgical procedures. Although clip 100 is described herein with respect to ligation, it is understood that a variety of other applications are possible as well. Clip 100 may have tissue grasping elements, elasticity-modifying elements, and open volume-creating elements, which create open volume to receive displaced material, as described previously herein.

Clip 100 can have any shape in its open configuration that allows it to effectively ligate a vessel, such as a substantially U-shaped or a substantially V-shaped design. As noted above, in an exemplary embodiment, the clip 100 is substantially U-shaped. That is, proximal portions 120a, 140a of the leg members 120, 140 of the clip 100 are oriented at an acute angle with respect to the central axis A of the clip 100, and transition at a knee portion 200, to an orientation where distal portions 120b, of the leg members 120, 140 are more nearly parallel with respect to one another and to longitudinal centerline 220.

Clip 100 comprises a compliant portion 110 and a rigid portion 105. One or both inner surfaces 120d, 140d may have a compliant portion 110 placed upon them. Compliant portion 110 may extend from apex 220 distally for a portion of the length of leg members 120, 140, as shown in FIG. 6. Alternatively, compliant portion 110 may extend the entire length from apex 220 to the distal ends of leg members 120, 140. Properties and dimensions of compliant portion 110 are chosen to have enough compliance fill gaps left by springback of clip 100, but to be less compliant than tissue to be ligated in order to compress the tissue. Properties and dimensions need not be uniform, for example, compliant portion 110 may be stiffer near apex 220 and more compliant, or compressible near the distal ends of leg portions 120, 140. Also, compliance may change as compliant portion 110 is compressed, for example, more compression may cause compliant portion 110 to stiffen. Compliant portion 110 can have properties, such as compressibility, of about 27.6 to 103.4 kPa (four to fifteen psi) at 10% to 75% compression. The thickness of compliant portion may be from about 0.254 mm (0.01 inch) to about 1.27 mm (0.05 inch). A designer may use materials and dimensions to cause compliant portion 110 to cooperate with rigid portion 105 to advantageously staunch blood flow within tissue to be ligated.

Compliant portion 110 may be created from biodegradable absorbable polymers that are synthetic or biologic derived. As an example, biodegradable synthetic absorbable polymers can include polydioxanon film sold under the trademark PDS ® or with a Polyglycerol sebacate (PGS) film or other biodegradable films from PGA (Polyglycolic acid, marketed under the trade mark Vicryl™), PCL (Polycaprolactone), PLA or PLLA (Polylactic acid), PHA (polyhydroxyalkanoate), PGCL (poliglecaprone 25, sold under the trademark Monocryl™), PANACRYL® (Ethicon, Inc., Comperville, NJ), Polyglactin910, Polyglyconage, PGA/TMC (polyglycolide-trimethylene carbonate sold under the trademark Biosyn®), polyhydroxybutyrate (PHB), poly(vinylpyrrolidone) (PVP), poly(vinyl alcohol) (PVA), or a blend of copolymerization of the PGA, PCL, PLA, PDS monomers. Suitable biologic derived materials may include but are not limited to platelet poor plasma (PPP), platelet rich plasma (PRP), starch, chitosan, alginate, fibrin, thrombin, polysaccharide, cellulose, collagen, bovine collagen, bovine pericardium, gelatin-resorcin-formalin adhesive, oxidized cellulose, mussel-based adhesive, poly (amino acid), agarose, polyetheretherketones, amylose, hyaluronan, hyaluronic acid, whey protein, cellulose gum, starch, gelatin, silk, or other material suitable to be mixed with biological material and introduced to a wound or defect site, including combinations of materials, or any material apparent to those of ordinary skill in the art in view of the teachings herein.

Rigid portion 105 of clip 100 can also have physical properties, such as yield strength, that are appropriate for a desired application. In an exemplary embodiment, the yield strength is greater than about 193 MPa (28 ksi) and less than about 413.7 Mpa (60 ksi), and more preferably in the range of about 206.8 MPa (30ksi) to 344.7 MPa (50 ksi). Rigid portion 105 of clip 100 is generally made of a malleable material that can be formed into a closed shape, but has residual elasticity that causes an amount of springback.

Rigid portion 105 of clip 100 disclosed herein can be made from a variety of surgically-appropriate materials including metals and polymers. Moreover, the material can be a bioabsorbable material or a non- bioabsorbable material. In one embodiment, the clip 100 can be made of a metal or a metal alloy having relatively high annealed state yield strength and a relatively high strain hardening rate, in comparison to existing ligation clips. Suitable metals include tantalum, titanium, stainless steel, or alloys thereof. By way of non-limiting example, the clip 100 can be made from commercially pure titanium or ASTM grade CP1 titanium, CP9 titanium, or CP5 titanium. This material, when compared with conventional materials, is able to be strain hardened to a greater extent without causing excessive gaps in the formed clip 100. Alternatively, the existence of compliant portion 110 allows for materials and geometry that cause more elasticity in rigid portion 105 of clip 100 than would otherwise be considered. Compliant portion 110 will fill gaps caused by elastic springback after clip formation to create a design more forgiving of material variations.

One skilled in the art will appreciate that the size of clip 100 can vary depending upon its particular application. In an exemplary embodiment, clip 100 can have a length *l* (similar to length il in FIG. 1) in the range of about 5 mm to 15 mm, and more preferably in the range of about 7.5 mm to 8.5 mm. In its open configuration, the clip 100 can have a width W, similar to width W shown in FIG. 3, between opposed inner surfaces 120d, 140d of the leg members 120, 140 in the range of about 2 mm to 8 mm, and more preferably in the range of about 3 mm to 4 mm. The size of the leg members 120, 140 can also vary depending upon the particular application, however in one embodiment, each leg member 120, 140 can have a width w, similar to width w shown in FIG. 2E, less than 1.27 mm (0.050 inch), more preferably in the range of about 0.635 mm(0.025 inch) to about 1.016 mm (0.040 inch), most preferably less than about 0.889 mm (0.035 inch). Moreover, each leg member 120, 140 can have a height H (similar to height H shown in FIG. 3) in the range of about 0.381 mm (0.015 inc)h to0.762 mm (0.030 inch), and more preferably in the range of about 0.4572 mm(0.018 inch) to 0.635 mm (0.025 inch), and most preferably in the range of about 0.4826 mm (0.019 inch) to 0.508 mm (0.020 inch).

Clip 100 is further designed so that, upon closure, a vessel, for example, is completely encased between the leg members 120, 140 of the clip 100. This is done by urging the leg members 120, 140 of the clip 100 together, typically with the assistance of an applier, to surround the vessel. A typical applier for clip 100 can be one as described in United States Patent Number 7,731,724 to Huitema et al.

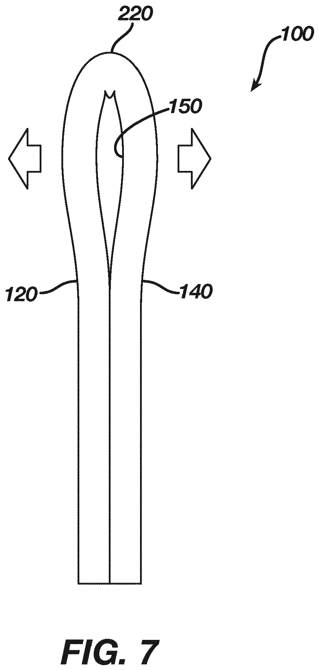

FIG. 7 shows a clip closed only at the distal end leaving a proximal opening 150 between the legs. The material used in rigid portion 105 has elasticity. After clamping the clip closed around tissue, residual elastic forces can cause the proximal portion of the clip to spring back and to open in the directions of the arrows in FIG. 7.

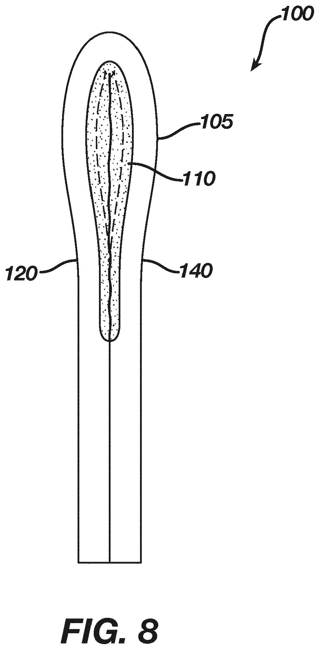

FIG. 8 depicts a closed clip 100 showing compliant portion 110 filling proximal opening caused by elasticity in rigid portion 105. Typically, a user would have an applier or forming tool with a clip 100 in the jaws. The user would place clip 100 over tissue to be ligated, such as a blood vessel, and cause the jaws of the applier to move together forcing leg members 120, 140 to move or deform towards each other. The deformation of clip 100 has a plastic component and an elastic component. The user of the applier continues to force leg members 120, 140 together until ligation of tissue is achieved and clip 100 is in the formed position. After formation of clip 100, release of the forming tool can cause leg members 120, 140 of rigid portion 105 to elastically move laterally, or spring back, causing separation of leg members 120, 140. The residual forces from the elastic portion of the deformation cause the leg members 120, 140 to separate the amount of elastic deformation, resulting in an opening 150. However, compliant portion 110 has enough thickness to fill any opening created when leg members 120, 140 separate. Clip 100 can be designed so that the thickness of compliant portion 110 is greater than the gap created by separation after clip formation, or so that the separation amount is less than the total of the thickness of tissue to be ligated and the thickness of compliant portion 110. Clip 100 can further be designed so that force placed upon a vessel by compliant portion 110 is sufficient to keep the vessel closed against the vessel's internal pressure, caused by, for example, blood attempting to flow through a ligated vein or artery. Also, compliant portion 110 may be designed to minimize forces against leg members 120, 140, to minimize separation after clip formation.

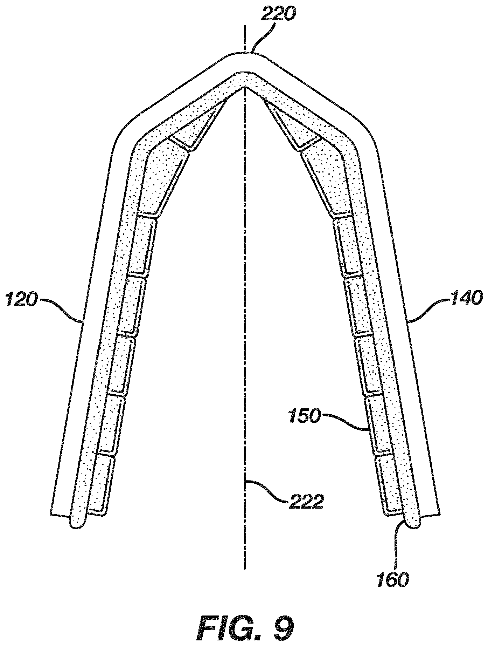

FIG. 9 shows compliant portion 110 having a plurality of ribs 150. Ribs 150 extend towards longitudinal centerline 222 from a compliant portion base 160 formed along at least one inner surface of inner surfaces 120d and 140d of rigid portion 105. Clip 100 may have a compliant portion 110 with at least one, and perhaps a plurality of ribs 150 extending from a base 160. Ribs 150 may be complementary in shape to each other to interlace upon closing, thus providing greater closure and gripping of tissue placed within leg members 120 of clip 100.

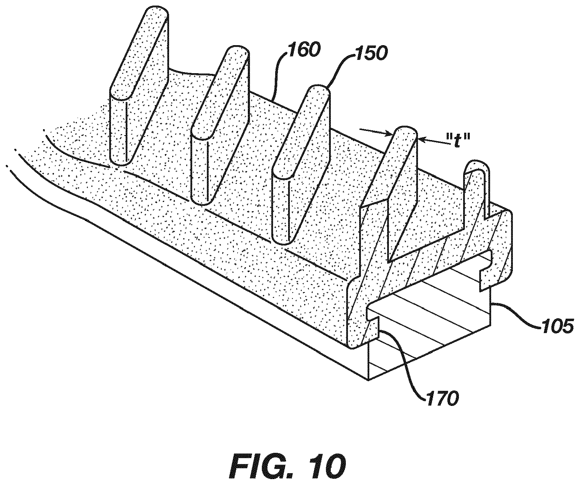

FIG. 10 shows in isometric view a set of ribs 150 that are angled to the longitudinal length of leg member 120, and that extend towards longitudinal centerline 222. Ribs 150 may be angled, parallel, or perpendicular to the longitudinal length of leg members 120, 140. Angling ribs 150 at different angles may serve to present different cross-sectional areas to tissue to apply optimum pressure to compliant portion 110 to cause optimum compression. Ribs 150 of FIG. 10 are shown having a constant thickness "t" from base 160 to the open ends of ribs 150. Thickness "t" can vary, however, from a thicker portion near base 160 to a thinner portion at the open end. Thickness "t" could also vary from a thinner dimension near base 160, becoming thicker near the open end, or other variations may occur to a designer of ribs 150.

FIG. 10 further shows in isometric view a groove 170 placed along leg members 120, 140. A portion of one leg member is shown, but groove 170 could be placed along one or both leg members 120, 140. Such a groove 170 can hold compliant portion 110 to rigid portion 105. Compliant portion 110 may be overmolded to rigid portion 105, for example, with the polymer flowing into groove 170 and hardening to hold compliant portion 110 to rigid portion 105. Groove 170 may be substantially rectangular, as shown, or it may be wider at the base to create a dovetail joint to more firmly hold compliant portion 110 to rigid portion 105 of leg members 120, 140.

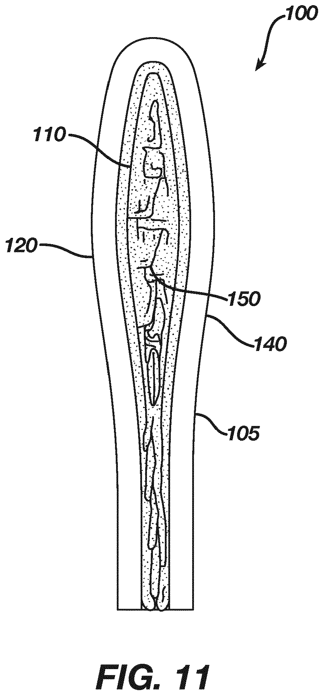

FIG. 11 shows clip 100 in the closed position, with a ribbed compliant portion 110. When clip 100 is in a closed position, ribs 150 can overlap and interlock to better grip and hold tissue between leg members 120 of clip 100. As another example, some ribs 150 may interlock, however, some ribs 150 may interfere upon closure of clip 100 to cause a desired pressure distribution on tissue to be ligated.

One skilled in the art will appreciate that features presented herein may be used advantageously to optimize holding and tissue compression of surgical clips. Thus, a compliant portion with or without ribs may be used with a clip having, for example, a raised portion, such as a raised portion 26a or 26b (FIG. 3) on an outside portion of one or more leg members 120. Additionally, clip 100 could have tissue contacting surfaces either on compliant portion 110 or rigid portion 105. Clip 100 could have a notch 24, such as notch 24 depicted in FIG. 4A, or tongue and groove configurations as depicted in FIG. 2B.

One skilled in the art will appreciate further features and advantages of the invention based on the above-described embodiments. Accordingly, the invention is not to be limited by what has been particularly shown and described, except as indicated by the appended claims.

## Claims

1. A surgical clip (100), comprising:
a. a pair of opposed first and second leg members (120,140) having proximal (120a,140a) and distal ends (120b,140b), said opposed first and second leg members deformable towards each other causing a clip deformation, said clip deformation having an elastic component and a plastic component, wherein said elastic component creates a proximal opening in a deformed clip after a forming tool is released; and
b. an apex (220) having opposed ends joining the proximal ends of said first and second leg members,
**characterized in that** the surgical clip further comprises:
a compliant portion (110) formed on an inner surface of at least one of said first and second leg members, said compliant portion having a thickness sufficient to fill said proximal opening.

2. The surgical clip of claim 1, wherein said surgical clip defines a longitudinal centerline (222), and said compliant portion comprises a plurality of ribs (150) extending away from said inner surface towards said longitudinal centerline.

3. The surgical clip of claim 2, wherein said plurality of ribs extends from said first and said second leg members, and at least a portion of said plurality of ribs interlocks when said surgical clip is in a formed position.

4. The surgical clip of claim 1, wherein said compliant portion is configured to be less compliant than tissue to which said surgical clip is applicable.

5. The surgical clip of claim 1, wherein said one of first and second leg members comprises a groove (170) extending along a portion thereof and said compliant portion is overmolded onto said one of first and second leg members utilizing said groove.

6. The surgical clip of claim 1, wherein said compliant portion is created from an absorbable material.

7. The surgical clip of claim 1, wherein said compliant portion is created from a material that swells in the presence of moisture.

8. The surgical clip of claim 1, wherein said compliant portion has a compliance that varies with the amount of compression undergone by said compliant portion.

9. The surgical clip of claim 1, wherein said compliant portion has a compliance that varies along a longitudinal length of said one of first and second leg members upon which said compliant portion is formed.

## Patentansprüche

1. Chirurgische Klammer (100), umfassend:
a. ein Paar gegenüberliegender erster und zweiter Schenkelelemente (120, 140) mit proximalen (120a, 140a) und distalen Enden (120b, 140b), wobei die gegenüberliegenden ersten und zweiten Schenkelelemente zueinander verformbar sind und eine Klammerverformung verursachen, wobei die Klammerverformung eine elastische Komponente und eine plastische Komponente aufweist, wobei die elastische Komponente eine proximale Öffnung in einer verformten Klammer erzeugt, nachdem ein Formwerkzeug freigegeben wird; und
b. einen Scheitelpunkt (220) mit gegenüberliegenden Enden, die die proximalen Enden der ersten und zweiten Schenkelelemente verbinden,
**dadurch gekennzeichnet, dass** die chirurgische Klammer ferner das Folgende umfasst:
einen nachgiebigen Abschnitt (110), der auf einer Innenfläche mindestens eines der ersten und zweiten Schenkelelemente ausgebildet ist, wobei der nachgiebige Abschnitt eine Dicke aufweist, die zum Ausfüllen der proximalen Öffnung ausreicht.

2. Chirurgische Klammer nach Anspruch 1, wobei die chirurgische Klammer eine Längsmittellinie (222) definiert und der nachgiebige Abschnitt eine Vielzahl von Rippen (150) umfasst, die sich von der Innenfläche weg zur Längsmittellinie erstrecken.

3. Chirurgische Klammer nach Anspruch 2, wobei sich die Vielzahl von Rippen von den ersten und zweiten Schenkelelementen erstreckt und zumindest ein Teil der Vielzahl von Rippen arretiert, wenn sich die chirurgische Klammer in einer geformten Position befindet.

4. Chirurgische Klammer nach Anspruch 1, wobei der nachgiebige Abschnitt dazu ausgelegt ist, weniger nachgiebig als Gewebe zu sein, an das die chirurgische Klammer anbringbar ist.

5. Chirurgische Klammer nach Anspruch 1, wobei das eine der ersten und zweiten Schenkelelemente eine Rille (170) umfasst, die sich entlang eines Abschnitts davon erstreckt und der nachgiebige Abschnitt als Umformung auf dem einen der ersten und zweiten Schenkelelemente unter Verwendung der Rille aufgebracht ist.

6. Chirurgische Klammer nach Anspruch 1, wobei der nachgiebige Abschnitt aus einem saugfähigen Material erzeugt wird.

7. Chirurgische Klammer nach Anspruch 1, wobei der nachgiebige Abschnitt aus einem Material erzeugt wird, das in Anwesenheit von Feuchtigkeit aufquillt.

8. Chirurgische Klammer nach Anspruch 1, wobei der nachgiebige Abschnitt eine elastische Nachgiebigkeit aufweist, die mit der Kompressionsmenge, dem der nachgiebige Abschnitt ausgesetzt ist, variiert.

9. Chirurgische Klammer nach Anspruch 1, wobei der nachgiebige Abschnitt eine elastische Nachgiebigkeit aufweist, die entlang einer longitudinalen Länge des einen der ersten und zweiten Schenkelelemente, auf dem der nachgiebige Abschnitt ausgebildet ist, variiert.

## Revendications

1. Pince chirurgicale (100), comprenant :
a. une paire de premier et second éléments de branche opposés (120, 140) ayant des extrémités proximale (120a, 140a) et distale (120b, 140b), lesdits premier et second éléments de branche opposés pouvant se déformer l'un vers l'autre, provoquant une déformation de la pince avec une composante élastique et une composante plastique, dans laquelle ladite composante élastique crée une ouverture proximale dans une pince déformée après qu'un outil de formation est dégagé ; et
b. un sommet (220) ayant des extrémités opposées joignant les extrémités proximales desdits premier et second éléments de branche,
**caractérisée en ce que** la pince chirurgicale comprend en outre :
une partie souple (110) formée sur une surface interne d'au moins l'un desdits premier et second éléments de branche, ladite partie souple ayant une épaisseur suffisante pour remplir ladite ouverture proximale.

2. Pince chirurgicale selon la revendication 1, ladite pince chirurgicale définissant une ligne médiane longitudinale (222), et ladite partie souple comprenant une pluralité de nervures (150) s'étendant depuis ladite surface interne vers ladite ligne médiane longitudinale.

3. Pince chirurgicale selon la revendication 2, dans laquelle ladite pluralité de nervures s'étend depuis lesdits premier et second éléments de branche, et au moins une partie de ladite pluralité de nervures se verrouille lorsque ladite pince chirurgicale se trouve dans une position formée.

4. Pince chirurgicale selon la revendication 1, dans laquelle ladite partie souple est configurée pour être moins souple que le tissu auquel ladite pince chirurgicale est applicable.

5. Pince chirurgicale selon la revendication 1, dans laquelle l'un des premier et second éléments de branche comprend une rainure (170) s'étendant le long d'une partie de celui-ci et ladite partie souple est surmoulée sur ledit élément parmi le premier et le second éléments de branche en utilisant ladite rainure.

6. Pince chirurgicale selon la revendication 1, dans laquelle ladite partie souple est créée à partir d'un matériau absorbable.

7. Pince chirurgicale selon la revendication 1, dans laquelle ladite partie souple est créée à partir d'un matériau qui se gonfle en présence d'humidité.

8. Pince chirurgicale selon la revendication 1, dans laquelle ladite partie souple a une souplesse qui varie avec la quantité de compression subie par ladite partie souple.

9. Pince chirurgicale selon la revendication 1, dans laquelle ladite partie souple a une souplesse qui varie le long d'une partie longitudinale dudit élément des premier et second éléments de branche sur lequel ladite partie souple est formée.
